**Europäisches Patentamt**

**European Patent Office**

**Office européen des brevets**

(19)

(11) Veröffentlichungsnummer : **0 192 996 B1**

# (12) EUROPÄISCHE PATENTSCHRIFT

(45) Veröffentlichungstag der Patentschrift :
13.12.89

(21) Anmeldenummer : 86101322.5

(22) Anmeldetag : 01.02.86

(51) Int. Cl.⁴ : **A 61 F   2/30**

(54) Gelenk-Endoprothese.

(30) Priorität : 01.03.85 DE 3507187

(43) Veröffentlichungstag der Anmeldung :
03.09.86 Patentblatt 86/36

(45) Bekanntmachung des Hinweises auf die Patenterteilung : 13.12.89 Patentblatt 89/50

(84) Benannte Vertragsstaaten :
AT BE CH DE FR GB IT LI LU NL SE

(56) Entgegenhaltungen :
DE—A— 3 336 005

(73) Patentinhaber : S + G IMPLANTS GMBH
Grapengiesserstrasse 21
D-2400 Lübeck (DE)

(72) Erfinder : Grundei, Hans
Gärtnergasse 4
D-2400 Lübeck (DE)
Erfinder : Biehl, Thomas, Dr.
Ismainger Strasse 22
D-8000 München 80 (DE)

(74) Vertreter : Wilcken, Thomas, Dipl.-Ing. et al
Musterbahn 1
D-2400 Lübeck (DE)

EP 0 192 996 B1

## Beschreibung

Die Erfindung bezieht sich auf eine Gelenk-Endoprothese mit Gelenk- und Schaftteil, die durch einen Adapter im Abstand voneinander veränderbar sind, wobei der Adapter aus zwei zueinander mit dem Schaftteil unverdrehbaren Hülsen besteht, von denen die eine einen axial unbewegbaren, von außen durch ein Werkzeug verdrehbaren Kopf aufnimmt, der mit einem Gewindeschaft in der anderen, mit Innengewinde versehenen Hülse verschraubbar ist.

Es hat sich gezeigt, daß die Verdrehung des mit Schraube versehenes Kopfes des Adapters nach der vorerwähnten Gelenk-Endoprothese durch Einführen eines außen erfaßbaren Steckers in Radialbohrungen des Kopfes oder eine von außen betätigbare, in ein Schneckenrad des Adapterkopfes eingreifende Schnecke wegen der unter Umständen aufzuwendenden Kräfte zu Schwierigkeiten führen kann und daß der Kopf des verdrehbaren Adapterteils auf dem ganzen Umfang mit Radialbohrungen für den Eingriff des Steckers versehen werden muß oder als Schneckenrad mit einer besonders gelagerten, eingreifenden Schnecke ausgebildet werden muß.

Die Aufgabe der Erfindung besteht darin, den inneren zur Verlängerung der Endoprothese dienenden, von außen zu betätigenden Adapter wesentlich zu vereinfachen.

Diese Aufgabe wird bei der eingangs erwähnten Gelenk-Endoprothese dadurch gelöst, daß der Kopf abgekehrt von seinem Gewindeschaft mit Werkzeugangriffsflächen versehen ist, mit denen das Ende eines durch eine Axialbohrung des Gelenkteiles einzuführenden Werkzeuges kraftschlüssig zum Eingriff bringbar ist.

Dadurch kann der Adapter einfach mit geringen Kosten hergestellt und auf einfache Weise zur Verlängerung der Gelenk-Endoprothese betätigt werden.

Die Erfindung wird nachstehend anhand der Zeichnung erläutert. Es zeigen:

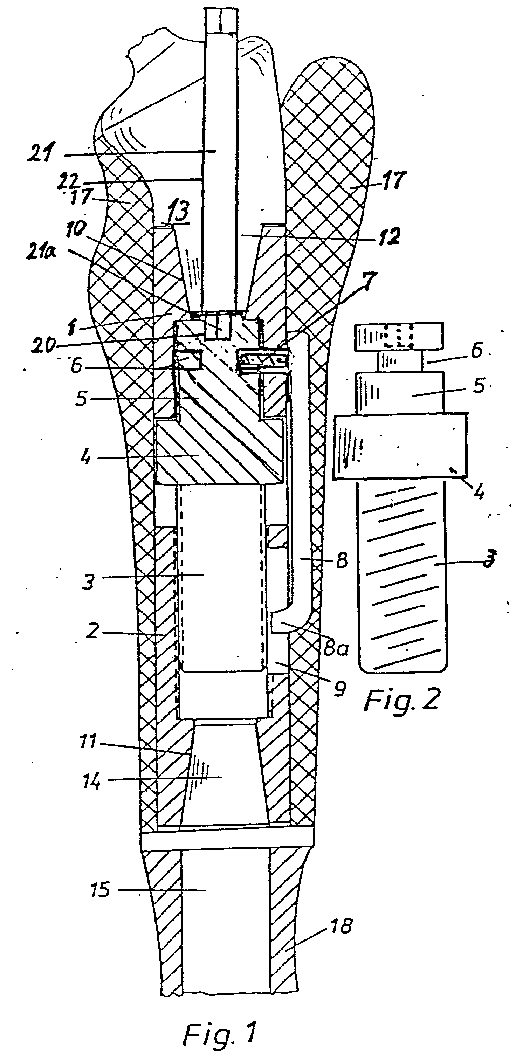

Figur 1 einen Achsschnitt durch eine zweiteilige Hüftgelenk-Endoprothese nach der Erfindung,

Figur 2 eine Seitenansicht des im Adapters nach Figur 1 zur Anwendung kommenden Kopfes mit Gewindeschaft.

Der Adapter nach der Erfindung besteht aus zwei zylindrischen, ausgerichteten Hülsen 1 und 2. In die Hülse 2 mit Innengewinde ist ein Gewindeschaft 3 einschraubbar, wobei der Kopf 4 zwischen den zugekehrten Enden der beiden Hülsen 1, 2 liegt und mit einer Verlängerung 5 in die Hülse 1 eingreift. Diese Verlängerung 5 ist mit einer Umfangsnut 6 versehen, in die eine durch die Hülsenwandung schraubbare Madenschraube 7 oder ein Stift eingreift, womit der Gewindeschaft 3 mit Kopf 4 in der Hülse 1 verdrehbar, aber axial nicht verstellbar gelagert ist.

Die Hülse 1 ist auf der Außenseite mit einer achsparallelen Strebe 8 starr verbunden, die mit einem abgekröpften Ende 8a in einen Langschlitz 9 der Hülse 2 eingreift und damit eine Verdrehung der beiden Hülsen 1, 2 zueinander verhindert.

Beide Hülsen 1, 2 sind an ihrem freien Ende je mit einem Innenkonus 10 und 11 versehen. In den Innenkonus 10 kann der Konus 12 eines Hüftgelenkprothesenteiles 13 formschlüssig eingreifen. In den Konus 11 der Hülse 2 kann der Konus 14 eines Prothesenschaftes 15 formschlüssig zum Eingriff gelangen. Es ist selbstverständlich möglich, die Hülse 1 mit einem Schaftteil zu verbinden und die Hülse 2 mit dem Femurteil eines Kniegelenkes oder die Hülse 1 mit dem Tibiateil einer Kniegelenkprothese und dann die Hülse 2 mit dem in das Schienenbein einzusetzenden Schaft zu verbinden.

Vor der Implantation der mit dem Adapter versehenen Endoprothese wird durch Verdrehung der Teile 3, 4 der Abstand der Hülsen 1, 2 eingestellt, so daß die Gesamtprothese auf die Länge des natürlichen zu ersetzenden Knochens abgestimmt ist, was aber auch noch nach der Implantation erfolgen kann, wobei der Adapter dem Längenteil des kranken, operativ zu entfernenden Knochenteiles angepaßt ist.

Der Prothesenteil 13 und die Hülse 1 werden in bekannter Weise noch mit einer diese Teile außen umgebenden Hülle 17 versehen, die den Raum ausfüllen soll, der vorher vom entfernten Knochenteil im Bein des Patienten eingenommen wurde. Auch die Hülse 2 kann von der Hülle 17 umgeben sein. An diese Hülle werden nach der Implantation die zugehörigen Muskelgewebe und Bänder angenäht, die vorher beim operativen Eingriff vom Knochen 18 gelöst wurden.

Um die Gelenk-Endoprothese an unterschiedliche Menschengrößen anpassen zu können, ist der Kopf 4 des Adapters an dem zu seinem Gewindeschaft abgekehrten Ende mit einer mittigen Ausnehmung 20 versehen, die ein flaches Profil (Schlitz) für den Eingriff eines Schraubenziehers oder eines sonstigen geeigneten Werkzeuges aufweist. Die Ausnehmung 20 kann auch das Profil eines Mehrkants zum Eingriff eines Mehrkantwerkzeuges besitzen.

Zur Anwendung des einen oder anderen Werkzeuges 21 ist der Gelenkteil 13 der Endoprothese mit einer axialen Durchbohrung 22 versehen, durch die ein Schraubenzieher oder ein stabförmiges Werkzeug 21 mit dem eingreifenden Mehrkant 21a hindurchgeführt werden kann. Zur Einführung des Werkzeuges 21 ist operativ ein Schnitt vorzunehmen, so daß dann das Oberende der Bohrung 22 frei für die Einführung des Werkzeuges 21 ist, welches am Oberende mit einer Handhabe erfaßt und verdreht werden kann.

Nach der Erfindung ist es selbstverständlich möglich, den Kopf 4 anstelle der Ausnehmung 20 mit einem Vorsprung zu versehen, mit dem ein Werkzeug mit entsprechender Ausnehmung seines Endes kraftschlüssig zum Eingriff bringbar ist.

## Patentansprüche

1. Gelenk-Endoprothese mit Gelenk- (13) und Schaftteil (15), die durch einen Adapter im Abstand voneinander veränderbar sind, wobei der Adapter aus zwei zueinander und zum Gelenkteil (13) unverdrehbaren Hülsen (1, 2) besteht, von denen die eine (1) einen axial unbewegbaren, von außen durch ein Werkzeug (21) verdrehbaren Kopf (4) aufnimmt, der mit einem Gewindeschaft (3) in der anderen, mit Innengewinde versehenen Hülse (2) verschraubbar ist, dadurch gekennzeichnet, daß der Kopf (4) des Adapters abgekehrt von seinem Gewindeschaft (3) mit Werkzeugangriffsflächen (20) versehen ist, mit denen das Ende (21a) eines durch eine Axialbohrung (22) des Gelenkteiles (13) einzuführenden Werkzeuges (21) kraftschlüssig zum Eingriff bringbar ist.

2. Gelenk-Endoprothese nach Anspruch 1, dadurch gekennzeichnet, daß das Werkzeug (21) ein von außen verdrehbarer Schraubenzieher (21) ist, dessen abgeflachtes Ende in eine angepaßte Ausnehmung (20) des Adapters greift.

3. Gelenk-Endoprothese nach Anspruch 1, dadurch gekennzeichnet, daß das Werkzeug (21) ein Mehrkantschlüssel ist, der am einen Ende von außen mittels einer Handhabe verdrehbar ist und am anderen Ende mit einem Mehrkantkopf (21a) in eine Mehrkantausnehmung (20) des Adapters greift.

## Claims

1. A joint endoprosthesis with a joint portion (13) and a shaft portion (15) of which the mutual spacing can be altered by means of an adaptor wherein the adaptor comprising two sleeves (1, 2) which are non-rotatable relative to one another and to the joint portion (13), of which the one (1) receives an axially immovable head (4) rotatable from the outside by means of a tool (21), which head can be screwed with a screw-threaded shaft (3) into the other sleeve (2) provided with an internal screw-thread, characterized in that on the side facing away from its screw-threaded shaft (3), the head (4) of the adaptor is provided with tool mating surfaces (20) with which the end (21a) of a tool (21) which is to be inserted through an axial bore (22) of the joint portion (13) can be brought into force-locking engagement.

2. A joint endoprosthesis according to claim 1, characterized in that the tool (21) is a screwdriver (21) turnable from the outside, the flattened end of which engages in a matching recess (20) of the adaptor.

3. A joint endoprosthesis according to claim 1, characterized in that the tool (21) is a polygonal key which is rotatable at one end from the outside by means of a handle and engages at the other end with a polygonal head (21a) in a polygonal recess (20) of the adaptor.

## Revendications

1. Endoprothèse pour articulation avec une partie d'articulation (13) et une partie de tige (15), qui sont distantes l'une de l'autre d'un espacement variable par un adaptateur, dans laquelle l'adaptateur est constitué de deux manchons (1, 2) immobilisés en rotation l'un par rapport à l'autre et par rapport à la partie d'articulation (13), et dont l'un (1) reçoit une tête (4), axialement immobile et destinée à être entraînée en rotation de l'extérieur à l'aide d'un outil (21), ladite tête (4) se visant par une tige filetée (3) dans l'autre manchon (2) muni d'un taraudage interne, caractérisée en ce que la tête (4) de l'adaptateur présente, du côté opposé à sa tige filetée (3), des surfaces (20) d'attaque d'outil, avec lesquelles l'extrémité (21a) d'un outil (21) introduit par un perçage axial (22) de la partie d'articulation (13) est destinée à être amenée en prise de façon à transmettre des forces par complémentarité de formes.

2. Endoprothèse pour articulation selon la revendication 1, caractérisée en ce que l'outil (21) est un tournevis (21) destiné à être entraîné en rotation depuis l'extérieur, et dont l'extrémité aplatie pénètre dans un évidement (20) adapté de l'adaptateur.

3. Endoprothèse pour articulation selon la revendication 1, caractérisée en ce que l'outil (21) est une clé polygonale qui, par une extrémité, est destinée à être entraînée en rotation de l'extérieur manuellement, et qui pénètre, à son autre extrémité, par une tête polygonale (21a) dans un évidement polygonal (20) de l'adaptateur.

EP 0 192 996 B1

*Fig. 1*

*Fig. 2*